# FASCICULE DE BREVET EUROPEEN

(11) **EP 2 501 809 B1**
(45) Date de publication et mention de la délivrance du brevet: **31.12.2014**
(21) Numéro de dépôt: 10800786.5
(22) Date de dépôt: 18.11.2010
(51) Int. Cl.: C12N 15/01, C12R 1/865, C12R 1/85, C12G 1/022, C12N 1/36, C12P 7/04, C12P 7/06, C12P 7/22, C12P 7/62, C12N 15/81

(54) **MÉTHODE POUR AMPLIFIER LA VOIE DES PENTOSES PHOSPHATE DANS DES SOUCHES DE LEVURES, LEVURES OBTENUES ET LEURS APPLICATIONS**
METHODEN ZUR AMPLIFIZIERUNG DES PENTOSE PHOSPHATE WEGES IN HEFE STÄMMEN, HEFEN HIERDURCH ERHALTEN UND DEREN VERWENDUNGEN
METHODS FOR THE AMPLIFICATION OF THE PENTOSE PHOSPHATE PATHWAY OF YEAST STRAINS, YEASTS OBTAINED AND THEIR APPLICATIONS

(30) Priorité: 20.11.2009 FR 0905585
(43) Date de publication de la demande: 26.09.2012
(73) Titulaire: Danstar Ferment AG, 6301 Zug (CH); Institut National de la Recherche Agronomique (INRA), 75338 Paris Cedex 07 (FR)
(72) Inventeur: DEQUIN, Sylvie, F-34000 Montpellier (FR); ORTIZ-JULIEN, Anne, F-31150 Cagnac sur Garonne (FR); CADIERE, Axelle, F-30640 Beauvoisin (FR)
(74) Mandataire: Peaucelle, Chantal
(86) Numéro de dépôt international: PCT/IB2010/055257
(87) Numéro de publication internationale: WO 2011/061702

(56) Documents cités:
- MILBRADT BIRGITTA ET AL: "Glucose-transport-deficient mutants of Schizosaccharomyces pombe: Phenotype, genetics and use for genetic complementation", MICROBIOLOGY (READING), vol. 140, no. 10, 1994, pages 2617-2623, XP002590956, ISSN: 1350-0872
- PEINADO ET AL: "Removing gluconic acid by using different treatments with a Schizosaccharomyces pombe mutant: Effect on fermentation byproducts", FOOD CHEMISTRY, ELSEVIER LTD, NL LNKD- DOI:10.1016/J.FOODCHEM.2006.11.070, vol. 104, no. 2, 3 mai 2007 (2007-05-03), pages 457-465, XP022055319, ISSN: 0308-8146
- CADIERE AXELLE ET AL: "The Saccharomyces cerevisiae zinc factor protein Stb5p is required as a basal regulator of the pentose phosphate pathway", FEMS YEAST RESEARCH, vol. 10, no. 7, 25 août 2010 (2010-08-25), pages 819-827, XP002620924,
- Sinha A. et al: "Induction of specific enzymes of the oxidative pentose phosphate pathway by glucono-delta-lactone in Saccharomyces cerevisiae", Journal of General Microbiology, vol. 138 1992, pages 1865-1873, XP055067337, GB Retrieved from the Internet: URL:http://mic.sgmjournals.org/content/138 /9/1865.full.pdf#page=1&view=FitH [retrieved on 2013-06-19]

## Description

L'invention a pour objet une méthode pour augmenter le flux à travers la voie des pentoses phosphate (VPP) et les levures ainsi obtenues. Elle vise également les applications de ces levures, notamment dans les industries de fermentation.

La VPP étant au centre du métabolisme carboné et redox de la levure, une amplification de cette voie présente un intérêt pour conduire à un remodelage métabolique important et ainsi conférer de nouvelles propriétés à la levure.

L'obtention d'une souche de levure ayant une VPP amplifiée peut être envisagée par différentes stratégies.

De nombreuses approches ont été développées ces dernières années dans le but d'obtenir des souches de *S. cerevisiae* capables de fermenter les pentoses. Ainsi, des souches de *S. cerevisiae* capables de fermenter le xylose en éthanol ont été obtenues par expression de gènes hétérologues permettant son assimilation (Eliasson *et al.,*2000). Cependant, la formation d'éthanol par ces souches reste limitée du fait de la sécrétion d'une partie du xylose consommé sous forme de xylitol L'accumulation du xylitol pourrait provenir de la limitation du flux à travers la partie non oxydative de la VPP dans *S. cerevisiae.* En effet, des études sur des mutants possédant une meilleure capacité à métaboliser le xylose ont montré une augmentation de l'activité de leur transaldolase ou leur transcétolase ou bien les deux (Becker et al., 2003; Sonderegger et al., 2005 ; Pitkanen et al., 2005). De plus, la délétion d'un des deux gènes (*ZWF1, GND1*) codant pour les enzymes NADPH dépendantes du segment oxydatif permet de diminuer également le rendement de production de xylitol et d'augmenter le rendement en éthanol (Eliasson *et al.,* 2000 ; Jeppsson *et al.,* 2002).

La sélection de mutants par évolution dirigée constitue une alternative aux approches d'ingénierie métabolique. Cette approche est basée sur la sélection de variants, obtenus par maintien d'une souche pendant de nombreuses générations en conditions sélectives pour forcer l'adaptation. Ce principe de sélection a été choisi par les inventeurs qui, de manière surprenante, sont parvenus à sélectionner des variants ayant accumulé des mutations permettant une amplification significative de la VPP. Ces mutations permettent de dégrader davantage de gluconate et confèrent aux souches des propriétés nouvelles, totalement inattendues (réduction de la production d'acétate, augmentation de celle d'esters, besoins en azote réduits).

L'invention a donc pour but de fournir une méthode d'obtention de mutants de levures dans lesquels la VPP est amplifiée par rapport à une souche parentale.

Elle vise également à fournir des mutants de levure dont le métabolisme, de manière inattendue, a été remodelé par rapport aux souches parentales, suite à l'amplification de la voie VPP.

L'invention vise en outre la mise à profit des propriétés de ces levures notamment dans les industries de fermentation.

La méthode d'obtention de souches mutantes de levures selon l'invention est caractérisée en ce qu'elle comprend la culture des souches sur un substrat à base de dérivé d'acide gluconique, plus particulièrement d'un sel d'acide gluconique (un tel sel étant désigné ci-après en abrégé par le terme « gluconate).

De manière avantageuse, le gluconate est incorporé dans la VPP au niveau de la 3^{ème} étape.

La culture des souches est effectuée afin de permettre leur adaptation dans des conditions de culture défavorables, sur au moins 10 générations, à une température d'incubation de 16°C à 32°C, de préférence de 28°C, sous agitation.

Les mutants ayant accumulé des mutations permettant une amplification de la VPP sont avantageusement sélectionnés par test de croissance et ceux ayant une densité optique (DO) supérieure à au moins 1,3 fois celle de la souche parentale sont récupérés.

L'invention vise également les mutants de souches de levures ayant une VPP améliorée par rapport à une souche parentale.

Ces mutants sont caractérisés en ce qu'ils sont susceptibles d'être obtenus par un procédé de culture comprenant les étapes
- d'adaptation d'une souche de levure, sur au moins 10 générations, en utilisant comme seule source de carbone un substrat à base de gluconate,
- de sélection des mutants ayant accumulé des mutations permettant une amplification de la VPP, ayant une DO finale d'au moins 1,3 fois supérieure à celle de la souche parentale non soumise aux conditions d'adaptation ci-dessus.

Plus spécialement, l'invention vise les mutants de souches de levures ayant, par rapport à une souche parentale, une VPP améliorée, une vitesse de fermentation supérieure,et par conséquent une consommation plus rapide de glucose, ainsi qu' une réduction de la production d'acétate

De manière avantageuse, les mutants de l'invention sont caractérisés par une forte production de composés aromatiques, tel l'alcool isoamylique, l'acétate d'isoamyle, le 2-phényléthanol.

Les souches de levures appartiennent de préférence au genre *Saccharomyces,* notamment aux espèces *cerevisiae, bayanus* ou à des genres non Saccharomyces.

Des souches préférées appartiennent au genre *Saccharomyces* et comprennent, notamment, les espèces *Saccharomyces cerevisiae, Saccharomyces bayanus, Saccharomyces uvarum* et *Saccharomyces kudriavzevii.*

D'autres souches préférées appartiennent au genre non *Saccharomyces.*

L'invention vise également les hybrides des souches définies ci-dessus.

L'invention vise en particulier la souche adaptée de *Saccharomyces cerevisiae* déposée à la Collection Nationale de Culture de Microorganismes (CNCM), 25 rue du Dr Roux , Paris, aux fins d'un dépôt selon le traité de Budapest, sous le n° CNCM I-4216, le 29 juillet 2009.

L'amplification de la VPP dans les souches mutantes de l'invention permet, notamment :
- une diminution de la production d'éthanol
- une diminution de la production d'acétate
- une modification de la production de certains composés d'arômes.
- une augmentation de la vitesse de fermentation

Ces propriétés présentent un intérêt majeur pour différentes utilisations de la levure, notamment dans les industries de fermentation.

D'autres caractéristiques et avantages de l'invention sont donnés dans les exemples qui suivent dans lesquels il est fait référence aux Figures 1 à 5, qui représentent, respectivement,
- les Figures 1 et 2 : les résultats de tests de croissance d'une souche issue d'une adaptation sur gluconate (I-4216) et de la souche parentale (I-4215), sur milieu YNB glucose (2%)(Figure 1) et sur milieu YNB gluconate (2%)(Figure 2);
- la Figure 3, la vitesse de fermentation de ces souches ;
- la Figure 4, la répartition des flux métaboliques dans une souche de l'invention et dans une souche parentale, et
- la Figure 5, les histogrammes représentant le gluconate résiduel dans le milieu.

### Adaptation dirigée et mode de culture

La souche industrielle I-4215 a été cultivée dans des tubes de 13 ml contenant 5 ml de milieu YNB gluconate [Yeast nitrogen base 0.67% DIFCO, et gluconate 2% (Sigma)]. Deux cultures ont été réalisées en parallèle. Les cultures sont ensemencées à une densité optique (DO) de 0,1, à partir d'une préculture d'une nuit sur YEPD (Bacto Yeast Extract 1% (Difco), Bacto Peptone 2% (Difco), glucose 2%). Dans le but d'éliminer toute trace de glucose, les cellules sont centrifugées et lavées avec de l'eau stérile. La culture est réalisée pendant de nombreuses générations, en diluant les cellules par transfert dans un nouveau milieu toutes les 3 générations (DO=0,8). Les cultures sont incubées à 28°C sous agitation (250 rpm). La croissance sur ce substrat étant très lente, 8 jours sont nécessaires pour atteindre une DO de 0,8.

### Analyse des souches adaptées

A intervalles réguliers, 200µL de cultures sont étalés sur une boîte contenant du milieu YEPD, afin de réaliser des tests de croissance permettant de mettre en évidence une évolution des souches. A cet effet, la croissance des souches issues de l'adaptation est comparée à celle de la souche parentale I-4215, sur YNB gluconate (2%) et sur YNB glucose (2%). Les cultures sont ensemencées à DO 0,1, à partir d'une pré-culture d'environ 14h sur YEPD. Les cultures sont réalisées dans des erlenmeyers de 250 ml contenant 50 ml de milieu et incubées à 28°C sous agitation (250 rpm).

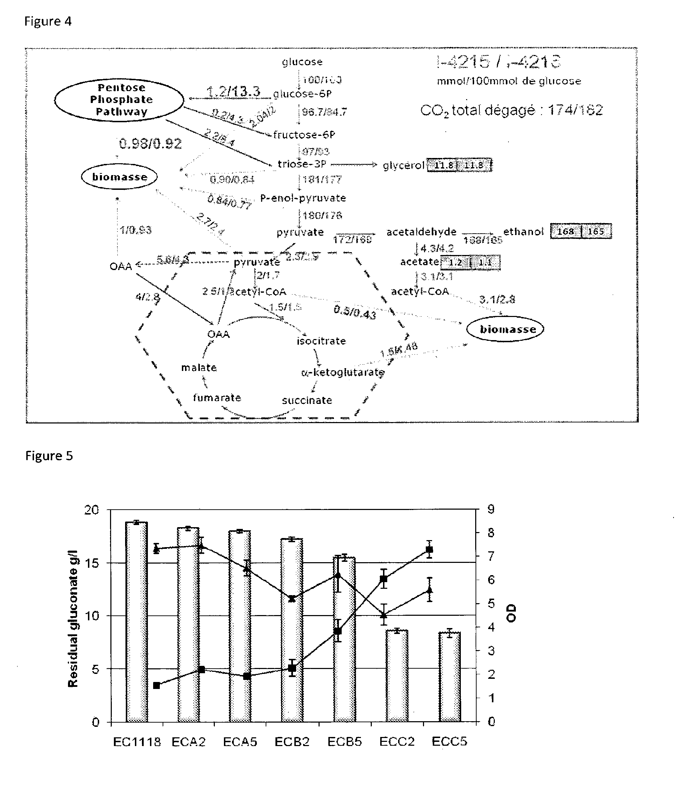

Après 23, 37 et 79 transferts correspondant à environ 70, 180 et 240 générations, des souches issues des deux adaptations ménées en parallèles ont été isolées sur l'évolution des capacités de croissance YNB gluconate (2%). Ces souches sont nommées ECA2 et I-4216 pour la sélection effectuée à 70 générations, ECB2 et ECB5 pour la sélection réalisée à 180 générations et ECC2 et ECC5 pour celle réalisée à 240 générations (Figure 5).

Leur capacité à consommer le gluconate est réprésentée sur la Figure 5 (carrés noirs : biomasse finale sur YNB gluconate ; triangles noirs : biomasse finale sur YNB glucose). Plus l'adaptation est prolongée, plus la capacité à consommer le gluconate augmente. En revanche, la capacité de ces souches à se multiplier sur glucose diminue plus l'adaptation est prolongée.

L'ensemble de ces souches a été testé dans des conditions de fermentation oenologique sur MS 70 contenant 240 g/l de glucose à 18°C. La souche I-4216 présente les meilleures capacités fermentaires (temps de fermentation réduit de 30%), alors que les souches ECB2 et ECC2 ne terminent pas leur fermentation. Les souches ECA2, ECB5 et ECC5 présentent un phénotype fermentaire intermédiaire entre I-4216 et I-4215. Une analyse comparative détaillée des propriétés souches I-4216 et I-4215 a alors été réalisée.

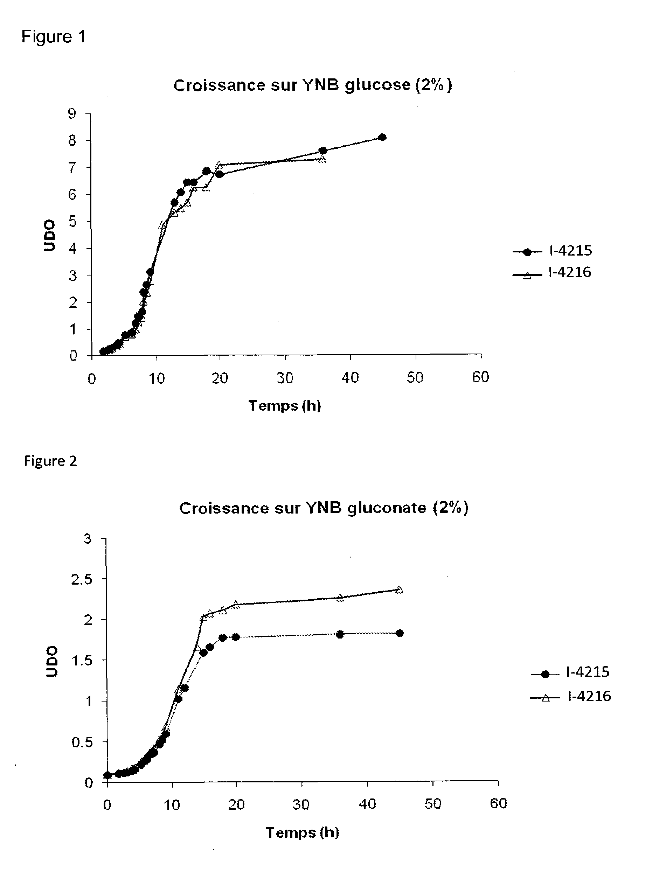

Aucune différence de croissance n'a été observée sur YNB glucose entre cette souche et I-4215 (Figure 1).

En revanche, sur YNB gluconate, la DO finale atteinte par I- 4216 est de 1,3 fois supérieure à celle atteinte par I-4215 (Figure 2).

### Propriétés fermentaires de la souche adaptée I- 4216

### Conditions de cultures

Les fermentations ont été réalisées dans des réacteurs de 1,2 litres (SGI, France) avec un volume réactionnel de 1 litre. Le milieu MS a été utilisé pour la pré-culture et la culture. Il s'agit d'un milieu synthétique qui simule un moût standard de raisin. Le milieu MS contient 20% glucose, 6 g/l d'acide malique, 6 g/l d'acide citrique, 100 mg/l d'azote, sous forme de NH₄Cl (79 mg/l) et d'acides aminés (28 mg/l). Le pH du milieu MS est ajusté à 3,3 avec une solution de NaOH à 32%. Des facteurs d'anaérobiose, ergostérol (7,5 mg/l), acide oléique (2,5 mg/l) et Tween 80 (0,21 g/l) sont ajoutés. Les pré-cultures ont été réalisées dans des erlenmeyers de 250 ml contenant 50 ml de milieu YEPD à 28°C sous agitation (150 rpm) pendant 8h. Ces pré-cultures ont été ensemencées à 0,1 UDO/ml à partir d'une pré-préculture sur YEPD réalisée pendant environ 14h. Les réacteurs ont été inoculés à partir des pré-cultures, à 0,5 million de cellules par ml et maintenus à température constante de 18°C avec une agitation permanente (300 rpm).

### Méthodes analytiques

### Méthodes analytiques

La croissance a été suivie par le comptage du nombre de cellules sur un appareil de type Coulter Counter (ZBI) à partir d'une fraction aliquote du milieu de culture.

Les métabolites ont été dosés dans le surnageant de culture, après centrifugation à 13000 rpm pendant 5 minutes. Les concentrations en glucose, glycérol, éthanol, pyruvate, succinate, acétate, α-cétoglutarate ont été déterminées par chromatographie liquide sous haute pression (HPLC) en utilisant une colonne de type HPX-87H (Bio-Rad).

Les concentrations en composés volatils (alcools supérieurs et esters) ont été déterminées par chromatographie en phase gazeuse.

### Résultats

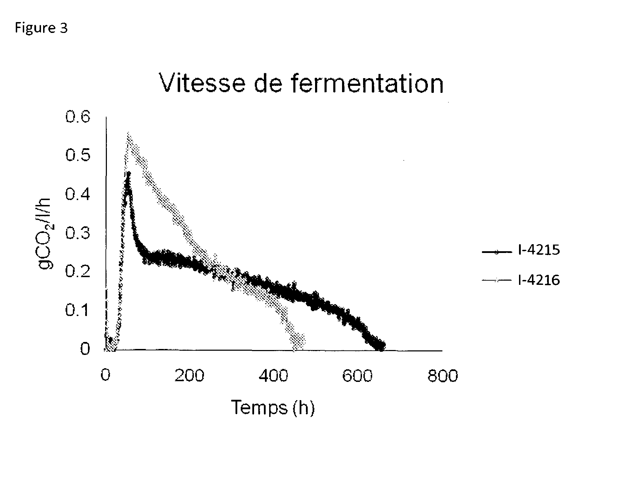

La souche I-4216 présente une vitesse maximale (Vmax) de fermentation supérieure à celle de I-4215, et consomme le glucose plus rapidement (Figure 3).

Le tableau 1 ci-dessous donne la balance carbone et redox des souches I-4215 et I-4216. Comme il ressort de l'examen de la figure 3, le résultat le plus marquant concerne la souche I- 4216 qui termine sa fermentation 200 heures plus tôt que la souche témoin. La souche adaptée présentant une biomasse finale inférieure à celle de la souche témoin (Tableau 1), la vitesse spécifique de fermentation est donc fortement augmentée par rapport à celle de I-4215

**Tableau 1**

| **Composé (g/l)** | I-4215 | I-4216 |
|---|---|---|
| Glucose | 239 ± 2 | 239 ± 2 |
| Biomasse | 2,1 ± 0,2 | 1,3 ± 0,2 |
| Ethanol | 119 ± 2,5 | 118 ± 4,0 |
| Glycérol | 7,4 ± 0,3 | 7,3 ± 0,7 |
| Pyruvate | 0,1 ± 0,0 | 0,1 ± 0,0 |
| Acétate | 0,70 ± 0,06 | 0,35 ± 0,01 |
| 2,3 butanediol | 0,4 | 0,6 |
| Succinate | 1,0 ± 0,1 | 0,9 ± 0,1 |
| | | |
| Balance Carbone (%) | 101 | 100 |
| | | |
| Balance redox (%) | 103 | 101 |

D'autre part, la souche adaptée produit moins d'acétate que I-4215. Cette réduction atteint un facteur 2 (tableau I)

L'acétate étant la seconde source de NADPH après la VPP, la diminution de la production d'acétate suggère que le flux à travers la VPP est augmenté dans ces souches.

L'obtention d'une souche à faible production d'acétate présente un intérêt en oenologie, notamment dans les vinifications de moûts blancs très clarifiés.

Aucun effet marqué n'est observé sur la production des autres sous produits. Les balances carbones et redox sont équilibrées dans les deux souches (Tableau 1).

La production des composés aromatiques a été analysée par chromatographie en phase gazeuse. Les résultats obtenus sur la production d'arômes sont résumés dans le tableau 2 ci-après.

**Tableau 2**

| | I-4215 | I-4216 |
|---|---|---|
| Isobutanol | 24,5 ± 1,4 | 29,5 ± 1,2 |
| Alcool isoamylique | 280,6 ± 13 | 415,4 ± 6,4 |
| 2-phényl éthanol | 90,2 ± 3,9 | 195,8 ± 0,6 |
| acétate d'isoamyle | 0,0 ± 0,0 | 12,4± 0,2 |

Des effets marqués ont été observés sur la production d'arômes de la souche adaptée par rapport à la souche témoin (I-4215).

La souche adaptée I-4216 présente une production accrue d'alcool isoamylique et d'acétate d'isoamyle (1,5 fois supérieur à EC1118) (Tableau 2). De plus, la production de 2-phényléthanol est plus élevée dans cette souche (2 fois) (Tableau 2). Le phényléthanol est produit à partir de la dégradation de la phénylalanine. Cet acide aminé est synthétisé à partir d'un intermédiaire de la VPP, l'érythrose-4-phosphate. Une augmentation de ce composé est cohérente avec une augmentation du flux à travers la VPP.

Une modification importante des propriétés aromatiques est ainsi observée, en partie expliquée par une augmentation du flux à travers la VPP.

Ces modifications présentent un grand intérêt en oenologie car l'acétate d'isoamyle et le 2-phényléthanol sont considérés comme des arômes positifs des vins et sont associés aux saveurs de banane et de poire pour le premier et de rose et de fleurs pour le second.

### Quantification du flux à travers la Voie des Pentoses phosphate chez la souche adaptée I-4216

L'ensemble des modifications observées montre que le flux à travers la voie des pentoses phosphate a été amplifié chez la souche adaptée I-4216. Aux fins de confirmation et de quantification de cet effet, une analyse comparative ¹³C flux chez I-4216 et I-4215 a été réalisée.

### Conditions de cultures

Les cultures sont réalisées dans des fioles à pénicilline contenant 10 ml de milieu synthétique dont la composition est modifiée pour qu'il contienne 100g/L de glucose dont 40% est marqué en ¹³C sur le C1 (Eurisotope) et 1.4 g/L de NH₄Cl comme seule source de carbone.
Les fioles sont ensemencées à 0,01 UDO.

### Traitement des cellules

A DO 3 (milieu de la phase exponentielle de croissance), les cellules sont centrifugées. L'incorporation du ¹³C dans les acides aminés est déterminée après hydrolyse acide de la biomasse et dérivatisation comme décrit par Gombert et al. (2001). Le surnageant de culture est conservé dans le but de déterminer la production de sous produits extracellulaires par HPLC.

### Méthode Analytique

La chromatographie en phase gazeuse couplée à la spectrométrie de masse (GC-MS) a été utilisée pour déterminer la distribution isotopique du carbone dans les acides aminés protéinogéniques obtenus au cours des cultures réalisées sur glucose ¹³C. La méthode utilisée est celle décrite par Christensen et Nielsen (1999). L'analyse est réalisée a l'aide d'une GC-MS de type GC-17A/GCMS-QP5050A (Shimadzu) fonctionnant en mode ionisation El (Electron Impact) et à 70 eV. La colonne utilisée lors des analyses est de type DB-170I (Lenght = 30 mm, ID = 250µm, film = 0,10µm) fournie par Agilent. Le spectromètre de masse fonctionne en mode SIM.

### Analyse et traitement des données

A partir des données brutes obtenues des mesures par GC-MS, le taux d'incorporation du ¹³C de chaque fragment est calculé de la manière suivante : SFL = 100 × [(1.*m*₁ + 2.*m*₂ + .... + n.*m*ₙ) × (*m*₀ + *m*₁ + *m₂* + ...+ *m*ₙ)⁻¹], *m*_{i>0} représente l'abondance des molécules contenant i carbones marqués.

De plus, la concentration des métabolites extracellulaires produits est dosée par HPLC.

L'ensemble de ces données (SFLs et métabolites extracellulaires) a été utilisé comme contrainte du modèle mathématique (Gombert et al. 2001) pour quantifier les flux dans le métabolisme carboné. 50 itérations sont réalisées et les répartitions de flux figurant sur la figure 4 sont la moyenne de 25 distributions choisies.

### Résultats

La distribution des flux, estimée en phase exponentielle de croissance, montre une augmentation considérable du flux à travers la VPP pour la souche I-4216. A ce stade, une légère diminution de la production d'éthanol est observée (168 and 165 mmol /100 mmol glucose pour I-4215 et I-4216 respectivement), tandis que le flux dirigé vers la biomasse est globalement diminué dans la souche I-4216 (figure 4). La perte de carbones résultant de l'amplification du flux à travers la VPP dans la souche ECA5 semble donc être compensée par une diminution la fois de la biomasse et de l'éthanol.

### Bilan de la fermentation de la souche I-4216 sur un moût de Chardonnay

### Conditions de cultures :

Les fermenteurs de 100 litres ont été ensemencés à 1 million de cellules par ml, à partir d'une pré culture en levain liquide des souches I-4215 et I-4216. Les milieux utilisés pour la fermentation et pour la préculture sont les mêmes (moût de chardonnay récolté en 2007). Une oxygénation de 15 mg/L ainsi qu'un ajout de di-ammonium hydrogéno-phosphate (30g/hl) ont été réalisés après la vitesse maximale. Méthodes analytiques :

Dosage de l'éthanol : dosage par densimètre de Paar. Cette méthode consiste à la distillation du vin alcalinisé par une suspension d'hydroxyde de calcium (98%) et à la mesure de la masse volumique du distillat. Pour cette mesure, le distillat est directement injecté dans un système oscillant dont la fréquence de vibration est modifiée par la masse de la substance injectée.

Mesure de l'acidité volatile : après distillation par entraînement à l'azote à 98°C, les acides du vin sont mis en présence d'un réactif coloré (le bleu de bromophénol) dont les variations d'intensité colorante sont proportionnelles à la teneur en acidité volatile du vin.

Mesure des composés d'arômes : les concentrations en composés volatils (alcools supérieurs et esters) ont été déterminées par chromatographie en phase gaz Headspace Agilent 6890 équipé d'un passeur HP 7694 et d'un détecteur FID.

Comme observé précédemment, I-4216 produit moins d'acidité volatile (dont l'acétate) que I-4215 (4 fois moins)(Tableau 3), produit beaucoup plus de composés aromatiques tels que l'acétate d'isoamyle (4 fois plus) (Tableau4).
Une diminution de la production d'éthanol dans la souche I-4215 est également observée. Elle se traduit par une diminution de son rendement éthanol de 0,47 à 0,46 g d'éthanol/g de sucre consommé (Tableau 3).

**Tableau 3**

| Echantillon | pH | Acidité g/L H₂SO₄ | Acidité volatile g/L H₂SO₄ | Alcool % vol | Glucose + Fructose g/l | Rendement éthanol g d'éthanol/g de sucre consommé |
|---|---|---|---|---|---|---|
| I-4215 | 3,3 ± 0,0 | 3,9 ± 0,1 | 0,4 ± 0,0 | 14,1 ± 0,0 | 0,8 ± 0,0 | 0,47± 0,00 |
| I-4216 | 3,3 ± 0,0 | 3,7 ± 0,1 | 0,1 ± 0,0 | 14,0 ± 0,0 | 1,6 ± 0,0 | 0,46± 0,00 |

**Tableau 4**

| | I-4215 | I-4216 |
|---|---|---|
| Isobutanol | 22.,5 ± 0,7 | 32,23 ± 1,5 |
| alcool isoamylique | 207,3 ± 6,9 | 250,4 ± 8,8 |
| acétate d'isobutyle | 0,0 ± 0,0 | 0,5 ± 0,0 |
| acétate d'isoamyle | 7,2 ± 0,1 | 33,6 ± 1,9 |
| butyrate d'éthyle | 0,3 ± 0,0 | 0,4 ± 0,0 |
| hexanoate d'éthyle | 0,3 ± 0,0 | 1,5 ± 0,1 |

### Références

Becker, J. and E. Boles (2003). "A modified Saccharomyces cerevisiae strain that consumes L-Arabinose and produces ethanol." Appl Environ Microbiol 69(7): 4144-50.
Blank, L. M., F. Lehmbeck, et al. (2005). "Metabolic-flux and network analysis in fourteen hemiascomycetous yeasts." FEMS Yeast Res 5(6-7): 545-58.
Christensen, B. and J. Nielsen (1999). "Isotopomer analysis using GC-MS." Metab Eng 1(4): 282-90.
Eliasson, A., C. Christensson, et al. (2000). "Anaerobic xylose fermentation by recombinant Saccharomyces cerevisiae carrying XYL1, XYL2, and XKS1 in mineral médium chemostat cultures." Appl Environ Microbiol 66(8): 3381-6.
Gombert, A. K., M. Moreira dos Santos, et al. (2001). "Network identification and flux quantification in the central metabolism of Saccharomyces cerevisiae under different conditions of glucose repression." J Bacteriol 183(4): 1441-51.
Jeppsson, M., B. Johansson, et al. (2002). "Reduced oxidative pentose phosphate pathway flux in recombinant xylose-utilizing Saccharomyces cerevisiae strains improves the ethanol yield from xylose." Appl Environ Microbiol 68(4): 1604-9.
Pitkanen, J. P., E. Rintala, et al. (2005). "Xylose chemostat isolates of Saccharomyces cerevisiae show altered metabolite and enzyme levels compared with xylose, glucose, and ethanol metabolism of the original strain." Appl Microbiol Biotechnol 67(6): 827-37.
Saint-Prix, F., L. Bonquist, et al. (2004). "Functional analysis of the ALD gene family of Saccharomyces cerevisiae during anaerobic growth on glucose: the NADP+-dependent Ald6p and Ald5p isoforms play a major role in acetate formation." Microbiology 150(Pt 7): 2209-20.
Sonderegger, M., M. Jeppsson, et al. (2004). "Fermentation performance of engineered and evolved xylose-fermenting Saccharomyces cerevisiae strains." Biotechnol Bioeng 87(1): 90-8.

## Revendications

1. Méthode d'obtention de souches mutantes de levures ayant une VPP (voie des pentoses phosphate) amplifiée, **caractérisée en ce qu'**elle comprend les étapes
- de culture des souches sur un substrat à base d'un sel d'acide gluconique,
- d'adaptation sur au moins 10 générations, à une température d'incubation de 16°C à 32°C, de préférence de 28°C, sous agitation,
- sélection des souches adaptées ayant accumulé des mutations permettant une amplification de la VPP, par test de croissance, sur les cultures ayant une DO supérieure à au moins 1,3 fois celle de la souche parentale.

2. Mutants de souches de levures ayant une VPP amplifiée par rapport à une souche parentale, **caractérisés en ce qu'**ils sont susceptibles d'être obtenus par un procédé de culture comprenant les étapes
. d'adaptation d'une souche de levure, sur au moins 10 générations, en utilisant comme seule source de carbone un substrat à base de gluconate,
. de sélection des mutants ayant accumulé des mutations permettant une amplification de la VPP, ayant une DO finale d'au moins 1,3 fois supérieure à celle de la souche parentale non soumise aux conditions d'adaptation ci-dessus, ces mutants présentant par rapport à une souche parentale, un temps de fermentation réduit d'au moins 30% et une production d'acétate réduite,d'un facteur d'au moins 2, une production accrue, de composés aromatiques, tel l'alcool isoamylique et l'acétate d'isoamyle, produits à un taux d'au moins 1,5 fois plus par rapport à la souche parentale, et de phényléthanol produit à un taux d'au moins 2 fois plus.

3. Mutants selon la revendication 2, **caractérisés en ce qu'**ils appartiennent au genre *Saccharomyces,* notamment aux espèces *cerevisiae, bayanus, uvarum* ou *kudriavzevii,* des genres non Saccharomyces, ou d'hybrides.

4. Souche mutante de *Saccharomyces cerevisiae* déposée à la Collection Nationale de Culture de Microorganismes (CNCM), 25 rue du Dr Roux , Paris, aux fins d'un dépôt selon le traité de Budapest, sous le n° CNCM I-4216, le 29 juillet 2009.

5. Application des souches selon l'une quelconque des revendications 2 à 4, en fermentation.

## Patentansprüche

1. Verfahren für den Erhalt von Mutantenstämmen von Hefen mit einem verstärkten PPW (Pentosephosphatweg), **dadurch gekennzeichnet, dass** es die Schritte umfasst:
- Kultivieren der Stämme auf einem Substrat auf Basis eines Gluconsäuresalzes,
- Adaptation über wenigstens 10 Generationen, bei einer Inkubationstemperatur von 16 °C bis 32 °C, vorzugsweise von 28 °C, unter Rühren,
- Auswahl der adaptierten Stämme, welche Mutationen angesammelt haben, die eine Verstärkung des PPW ermöglichen, mittels Wachstumstest an den Kulturen, die eine OD aufweisen, die wenigstens 1,3 mal größer ist als diejenige des Elternstammes.

2. Mutanten von Hefestämmen mit einem im Vergleich zu einem Elternstamm verstärkten PPW, **dadurch gekennzeichnet, dass** sie durch ein Kulturverfahren erhältlich sind, das die Schritte umfasst:
• Adaptation eines Hefestammesüber wenigstens 10 Generationen, indem als einzige Kohlenstoffquelle ein Substrat auf Basis von Gluconat verwendet wird,
• Auswahl der Mutanten, die Mutationen angesammelt haben, die eine Verstärkung des PPW ermöglichen, mit einer finalen OD, die wenigstens 1,3 mal größer ist als diejenige des nicht den obigen Adaptationsbedingungen unterzogenen Elternstammes, wobei diese Mutanten im Vergleich zu einem Elternstamm eine um wenigstens 30 % reduzierte Fermentationszeit und eine um einen Faktor von wenigstens 2 reduzierte Acetatproduktion, eine erhöhte Produktion von aromatischen Verbindungen, wie Isoamylalkohol und Isoamylacetat, die im Vergleich zu dem Elternstamm in einem wenigstens 1,5-mal höheren Anteil erzeugt werden, und von Phenylethanol, der in einem wenigstens 2-mal höheren Anteil erzeugt wird, aufweisen.

3. Mutanten nach Anspruch 2, **dadurch gekennzeichnet, dass** sie zu der Gattung *Saccharomyces*, insbesondere zu den Spezies *cerevisiae*, *bayanus*, *uvarum* oder *kudriavzevii*, Nicht-Saccharomyces-Gattungen, oder Hybriden gehören.

4. Mutantenstamm von *Saccharomyces cerevisiae*, hinterlegt bei der Collection Nationale de Culture de Microorganismes (CNCM), 25 rue du Dr Roux, Paris, zum Zwecke einer Hinterlegung gemäß dem Budapester Vertrag, unter der Nummer CNCM I-4216, am 29. Juli 2009).

5. Anwendung der Stämme nach einem der Ansprüche 2 bis 4, in Fermentationsverfahren.

## Claims

1. A method for obtaining mutant yeast strains having an amplified PPP, **characterized in that** it comprises the steps of:
- culturing the strains on a substrate based on a gluconic acid salt,
- adaptation over at least 10 generations, at an incubation temperature of from 16°C to 32°C, preferably of 28°C, with shaking,
- selecting the adapted strains having accumulated mutations allowing amplification of the PPP, by means of a growth test on cultures having an OD greater than at least 1.3 times that of the parental strain.

2. Yeast strain mutants which have an amplified PPP compared with a parental strain, **characterized in that** they are capable of being obtained by means of a method of culture comprising the steps of:
. adapting a yeast strain, over at least 10 generations, using as sole carbon source a substrate based on gluconate,
. selecting the mutants having accumulated mutations allowing amplification of the PPP, having a final OD at least 1.3 times greater than that of the parental strain not subjected to the above adaptation conditions, these mutants presenting, compared with a parental strain, a fermentation time reduced by at least 30% and an acetat production reduced by at least a factor of 2,
an increased productionof aromatic compounds, such as isoamyl alcohol and isoamyl acetate, produced at an at least 1,5 times higher rate compared to a parental strain, or phenylethanol, produced at an at least 2 times higher rate.

3. The mutants as claimed in claim 2, **characterized in that** they belong to the *Saccharomyces* genus, in particular to the species *cerevisiae, bayanus, uvarum* or *kudriavzevii, non-Saccharomyces* genera, or hybrids.

4. The mutant strain of *Saccharomyces cerevisiae* deposited at the Collection Nationale de Culture de Microorganismes (CNCM) [French National Microorganism Culture Collection], 25 rue du Dr Roux, Paris, for the purposes of a filing according to the Treaty of Budapest, under No. CNCM 1-4216, on July 29, 2009.

5. The use of the strains as claimed in any one of claims 2 to 4, in fermentation.
